# EUROPEAN PATENT APPLICATION

(11) **EP 0 682 928 A1**
(43) Date of publication of application: **22.11.1995**
(21) Application number: 94114286.1
(22) Date of filing: 12.09.1994
(51) Int. Cl.: A61F 13/15, A61F 5/44, A61F 13/56

(54) **Adjustable diapers particularly for adults**

(30) Priority: 09.05.1994 IT MI940336 U
(71) Applicant: TEKMA S.r.l., I-26010 Fiesco (CR) (IT)
(72) Inventor: Mandotti, Pierangelo, Scannabue CR (IT)
(74) Representative: Savi, Alberto

(57) **Abstract**

A pants diaper which include an external layer made by impermeable material, and an internal layer made by absorbing material including, in correspondence with one extremity, a couple of fins (7,7') made by elasticized material having, close to the external edge, a layer of adhesive (8,8') and including, on the opposite side, a couple of lateral edges (9,9') used in order to make stick to them the adhesive part of these fins (7,7'), being impressed, on these edges (9,9') the corresponding references, both to a different user's waist so that it is possible to make the diaper assume the final shape of a pants diaper of the required size.

## Description

The present invention proposes an adjustable diaper, particularly for incontinents, which is characterized by the fact that it presents, on one side, a couple of fins made by elasticized material having an adhesive and, on the opposite side, an edge on which are preprinted various sizes, corresponding to the different sizes of the user, and to which the extremities of the elasticized fins cling to, in order to realize a pant diaper, which is easier to be worn by the user.

Consequently, the particularity of the diaper, according to the invention, is the structure of the parts tending to increase remarkably its feasibility of use.

The invention is inserted in the field of diapers, particularly diapers for adults.

The diapers actually known can be divided into two big categories and, precisely, simple diapers and pants diapers.

The first ones include a layer of absorbing material, of appropriate thickness, applied to a layer made by impermeable material, generally polyethylene, having at the extremities adhesive flaps allowing the fixing in position after the application. The pants diapers are instead formed by a wear, still made by polyethylene or similar, having, on the bottom, an adequate absorbent.

The diaper of both types are produced in many shapes, depending on the kind of use and different necessities.

Generally the pants diapers are much easier to be worn, especially by an aged person who has a certain difficulty of movements.

In comparison with this advantage this kind of diapers present the disadvantage of a higher cost and the necessity of having in the store diapers of all sizes, in order to satisfy the clients.

Simple diapers are instead cheaper, they can be produced in only one size, but they are difficult to be worn particularly by aged people for whom the control of movements is often difficult. For these reasons it is sensed, in the field, the necessity of having diapers which can be worn with the same easiness peculiar to the pants diapers, but which, unlike these ones can be adapted to users of different sizes and economical to be produced.

This problem is solved by the diaper, according to the invention, which is characterized by the fact that it presents on one side a couple of elasticized fins, having at the extremities adhesive edge and, on the opposite side, edges on which are printed the references corresponding to the different sizes of the user, in order to realize a pants diaper of the suitable size, simply by making the adhesive flaps stick, in correspondence with references showing the size of the user.

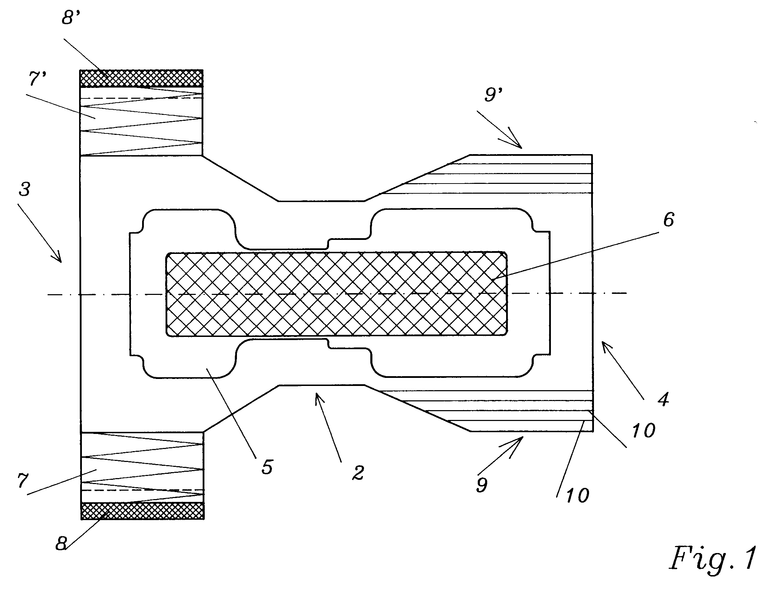

The present invention will be better understood thanks to the following detailed description, given as a non limitative example, with reference to the only figure enclosed, representing the top view of an open diaper, according to the invention. In figure N° 1 it is shown the paper made by impermeable material, particularly polyethylene, which constitutes the external layer of the diaper and which is shaped in such a way that it presents a central part of shorter lenght, indicated with 2, and a first and second part with extremities of longer lenght, indicated respectively with 3 and 4.

Paper 1 is shaped in such a way that it presents, in the central zone, a basin 5 in which a "fluff" or layer made by absorbing material 6 is inserted.

All this is then covered by a layer made by a suitable material, such as for exemple fabric non-woven.

In correspondence with a first extremity the diaper has, at the two ends, a couple of fins 7 and 7', made by elasticized material, having, in correspondence with the external edge, the same number of layers made by adhesive material, indicated with 8 and 8'. On the opposite side, in correspondence with the second extremity 4, the diaper has 2 edges, 9 and 9', on which a series of references are printed, indicated by 10, corresponding to the same sizes of the user's waist.

In short, by refolding the diaper and making the adhesive strips 8 and 8' stick to edges 9 and 9,' in correspondence with the reference corresponding to its own waist, it can be obtained a pants diaper of one's own size, ready to be worn.

Once the user knows the size of his own waist, consequently, he can easily use the diaper according to the invention since he has only to refold it and make fin 8 stick with edge 9 and fin 8' with edge 9'.

Bringing the strip of the adhesive material till in correspondence with the sign corresponding to the size of one's own waist. With this system, the user realizes a pants diaper of his own size, starting from a simple diaper of standard dimensions. This refolding operation of the diaper is of course made before wearing it, so that even a person who has not a perfect control of her own movements can prepare the pants diaper and wear them easily, without the necessity of being helped by other people.

The fins made by elasticized tissue provide to maintain the diaper adherent to the user's body.

In this way, it can be obtained a diaper which has the advantages of both models actually known, that is to say, the simple diaper and the pants diaper.

In fact it has an economical realization, it can be produced in only one or in a very small number of sizes since it can be adapted to the different sizes of the user and it is easy to be worn, assuming the definitive shape of a pants diaper.

Obviously both dimensions and materials used, can vary depending on the necessities of use.

## Claims

1. A pants diaper of the type including an external layer made by impermeable material, and an internal layer made by absorbing material, characterized by the fact that in correspondence with one extremity, it has a couple of fins made by an elasticized material, having close to the external edge, a layer of adhesive and, on the opposite side, a couple of lateral edges used for making adhere to them the adhesive part of these fins, on these edges, having impressed references corresponding, both, to a different user's waist, so that it is possible to make the diaper assume, the final shape of pants diaper of the required size.

2. A diaper as described and shown.
